# EUROPEAN PATENT APPLICATION

(11) **EP 1 820 529 A1**
(43) Date of publication of application: **22.08.2007**
(21) Application number: 06110071.5
(22) Date of filing: 17.02.2006
(51) Int. Cl.: A61M 16/10, A61M 16/08

(54) **Gas processing unit**

(71) Applicant: General Electric Company, Schenectady, NY 12345 (US)
(72) Inventor: Laurila, Santtu, 00500 HELSINKI (FI); Ranta, Janne, 02710 Espoo (FI); Tamminen, Anne, 00700 Helsinki (FI); Numerla, Maija, 00510 Helsinki (FI)
(74) Representative: Valkeiskangas, Tapio Lassi Paavali

(57) **Abstract**

The invention relates to gas processing unit in patient circuits for use in anaesthesia or respiratory care. The patient circuit comprises a gas source for creating gas mixture for patient breathing and circulating arrangement for delivering the gas mixture to the patient. The circulating arrangement comprises tubes through which gases flow to the patient and from the patient. The circulating arrangement has a proximal end connected to the gas source for creating gas mixture, and a distal end connected to the patient, and a processing unit. The processing unit comprising a housing and at least one processing element placed in the housing. The processing element (9,11) is arranged to form an angle other than 90 degrees with respect to a direction of gas flow in the tubes (5).

## Description

The invention relates to gas processing unit in patient circuits for use in anaesthesia or respiratory care, the patient circuit comprising a gas source for creating gas mixture for patient breathing and circulating arrangement for delivering the gas mixture to the patient, the circulating arrangement comprising tubes through which gases flow to the patient and from the patient, and the circulating arrangement having a proximal end connected to the gas source for creating gas mixture, and a distal end connected to the patient, and a processing unit, the processing unit comprising a housing and at least one processing element placed in the housing.

As described above the present invention relates to gas processing in patient circuits, for example to gas filtration in patient circuits for use in anaesthesia or respiratory care. A gas processing element, for example a filter is normally used in a patient breathing circuit to protect the patient and/or protect the equipment. Filters were originally employed to prevent the contamination of those parts of the apparatus which were the most difficult to clean or sterilize. They would typically be positioned at the expiratory port of the ventilator. However, as the use of disposable heat and moisture exchanger with a filter (HMEF) became popular the ideal position for these devices was interposed between the breathing circuit and the catheter mount providing "patient-end" filtration and effectively isolating the patient from the rest of the circuit. Patient close filters are placed between the circuit Y-piece and the patient. In low-flow anesthesia a filter will also protect the patient from inhaling dust from the CO₂ absorber.

Two different filtration techniques are available in the market, mechanical or electrostatic. Both types of filter are applied in the intensive care and anesthesia areas. They can both be used as machine close filters or patient close filters. It is common to use a mechanical filter in machine close application. Both are essentially a weave of synthetic fibers, but whereas a pleated membrane has an extremely close-woven mesh, thus optimizing the direct interception of microorganisms, an electret felt has larger gaps between the individual fibers.

During respiration, oxygen is transported from the surrounding air through the pulmonary system to the alveolus. There, oxygen and carbon dioxide exchange is taking place between the blood and the air in the lungs. To maintain an efficient pulmonary function we need to have a humid and warm climate in our lungs. During normal breathing, the respiratory tract is doing an excellent job to heat and moisturize air that enters the airways. For example, dry air on a cold winters day can be inhaled without any danger for the pulmonary system since the nose is such a good heat and moisture exchanger. Behind the point that is called the Isothermic Saturation Boundary (ISB), the incoming air has reached alveolar conditions and the climate is held constant. This boundary is during normal breathing placed approximately 5 cm below carina.

The air we breathe contains moisture and the amount of moisture depends on the relative humidity (RH) in the air. Ventilator/respirator during respirator care is connected to a distal patient device that is connected to a patient. When air enters the upper trachea during normal breathing it usually has a temperature of 33°C and a relative humidity of about 75%. The gases coming from the ventilator has a temperature of 20°C and a relative humidity of close to 0%. The reasons to have this cold and dry air in the ventilator are to prevent corrosion in the machine parts and avoid bacterial growth in humid air. Because of the very dry and cold gases, the ventilator air should be humidified and heated by an external humidifier, which will replace the function of the nose.

Conventional patient circuit filters with passive humidifiers (artificial noses), i.e. the filter and humidifier units known in the prior art have flat disc shaped filter and humidifier elements perpendicular to patient circuit. The wording "perpendicular to patient circuit" means that the filter and humidifier element is arranged to for an angle of essentially 90 degrees with respect to flow of gases in the patient circuit tubes. Because of this design the filter housing typically has to have a diameter much higher than the diameter of the breathing circuit in order to achieve reasonably low flow resistance (high filtering area) with a good filtering performance and moisture output with adequate amount of element. Unfortunately, with a high diameter, the product becomes bulky and the usability is compromised. Big filter housings create weight and strain to the patient circuit causing strain to the patient. They can also cause damage to the skin of the patient and hinder clinicians to have a complete access to the face of the patient. Furthermore, the total filtering area is not completely utilized in conventional filters because the flow is not passing the filter element uniformly due to the filter geometry.

The object of the invention is to obtain a gas processing unit by which the disadvantages of the prior art can be eliminated. This is achieved with the present invention. The gas processing unit of the invention is characterized in that the processing element is arranged to form an angle other than 90 degrees with respect to a direction of gas flow in the tubes.

An advantage of the invention is its simplicity whereby the invention can be materialized with low costs. The invention is also very flexible, i.e. the structure can be modified in various ways according to the existing needs, and still first class filtration efficiency is obtained.

The invention will now be described in greater detail with reference to the embodiments of the invention described in the attached drawing, of which
Figure 1 shows schematically a conventional patient breathing circuit with a conventional gas processing unit,
Figure 2 shows a conventional gas processing element used in the arrangement shown in Figure 1,
Figure 3 shows one embodiment of the invention,
Figure 4 shows a second embodiment of the invention,
Figure 5 shows a third embodiment of the invention,
Figure 6 shows a fourth embodiment of the invention,
Figure 7 shows a structure used for example in the embodiment shown in Figure 5, and
Figure 8 shows the principles how the pores distribute in the structure shown in Figure 7.

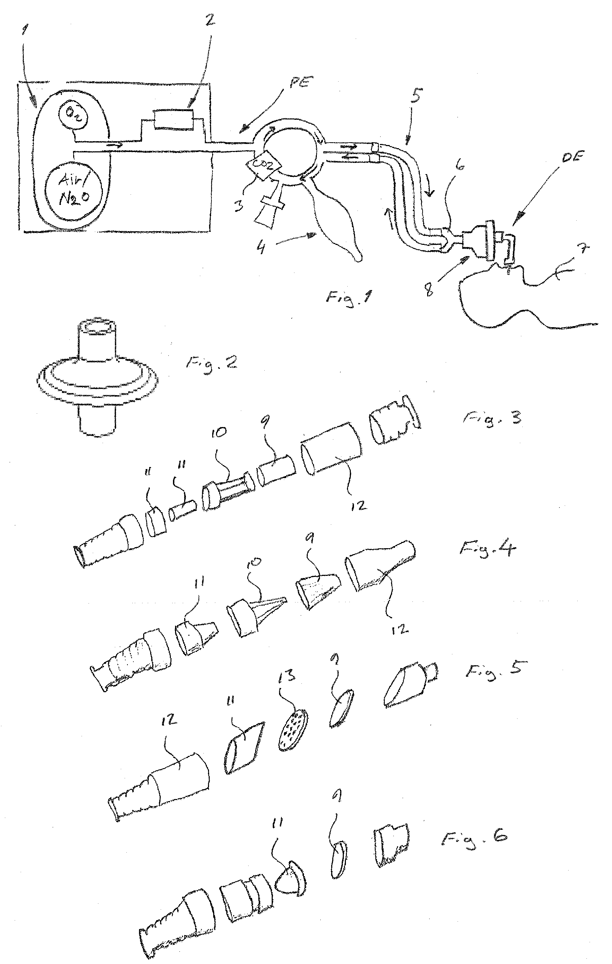

Figure 1 shows schematically a conventional patient breathing circuit. Reference number 1 shows generally fresh gas feeding means a reference number 2 an anaesthesia vaporizer. For example fresh gas feeding means 1 and vaporizer 2 form together a gas source for creating gas mixture for patient breathing. In this connection it is important to realize that the arrangement shown in Figure 1 is only one embodiment, i.e. the gas source can comprise only fresh gas source etc. Reference number 3 shows a CO2 absorber and reference number 4 a manual bag or a ventilator. Reference number 5 shows generally tubes for inhalation and expiration gases. Reference number 6 shows a Y-piece and reference number 7 shows a patient. Reference number 8 show a filter and humidifier unit. Reference PE shows a proximal end of the circuit described and reference DE shows a distal end of the circuit described.

Structural details and operating principles of the arrangement shown in Figure 1 are quite familiar to a person skilled in the art, and therefore said matters are not described in detail in this connection.

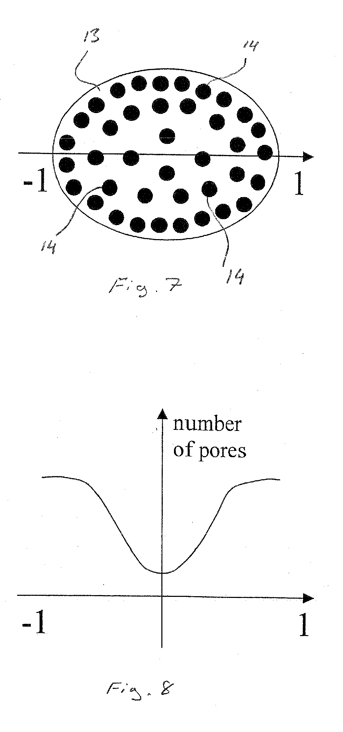

Figure 2 shows a conventional circuit filter used for example in the arrangement shown in Figure 1. Conventional patient circuit filters presented in the Figure 2 with passive humidifiers (artificial noses) have flat disc shaped filter and humidifier elements perpendicular to patient circuit. As told earlier the wording "perpendicular to patient circuit" means that the filter and humidifier element is arranged to for an angle of essentially 90 degrees with respect to flow of gases in the patient circuit tubes. This arrangement is clearly shown in Figure 1. Because of this design the filter housing typically has to have a diameter much higher than the diameter of the breathing circuit tubes in order to achieve reasonably low flow resistance (high filtering area) with a good filtering performance and moisture output with adequate amount of element. Unfortunately, with a high diameter, the product becomes bulky and the usability is compromised. Large and clumsy filter housings create weight and strain to the patient circuit causing strain to the patient. They can also cause damage to the skin of the patient and hinder clinicians to have a complete access to the face of the patient. Furthermore, the total filtering area is not completely utilized in conventional filters because the flow is not passing the filter element uniformly due to the filter geometry.

New geometries are introduced in the invention to obtain improved solutions for filtration and humidification for use in anaesthesia or respiratory care. Different filter geometries are applied to obtain smaller outside diameter of the filter housing while the filtration efficiency remains. The processing element is arranged to form an angle other than 90 degrees with respect to a direction of gas flow in the tubes. The term "a direction of gas flow" refers to the direction of the main gas flow in the tubes, i.e. to the direction in which the gas flow through the tubes. The angle mentioned above can be for example between 2 - 68 degrees, preferably between 7,5 - 60 degrees.

In other words, the diameter of the filter housing is minimized while the filter surface area is maximized without compromising the properties of the filter or patient's safety. The filtering element of this invention can be shaped as a cylinder or a cone by wrapping the filtering media around a supporting frame as is illustrated in Figures 3 and 4 or it can be a plane filter at an oblique angle as presented in the Figure 5.

Figure 3 shows one embodiment of the gas processing unit of the invention. In this embodiment the gas processing element comprises a filter element and a humidifier element. Reference number 9 shows a filter element that is shaped as a cylinder by wrapping the filtering media around a supporting frame 10. The humidifier element 11 may consist of several parts instead of one or humidifier may as well be wrapped around the filter element. All these elements are placed inside a housing 12.

Figure 4 shows the second embodiment of the invention. In this embodiment also the gas processing element comprises a filter element and a humidifier element. Reference number 9 shows a filter element that is shaped as a cone by wrapping the filtering media around a supporting frame 10. Reference number 11 shows a humidifier element. The humidifier element may as well be wrapped around the filter element. All these elements are placed inside the housing 12.

Figure 5 shows a third embodiment of the invention. In this embodiment the gas processing element comprises a filter element and a humidifier element. Reference number 9 shows a filter element. In this embodiment the filter element is a plane filter at an oblique angle with respect to the longitudinal axis of the structure, i.e. with respect to the flow direction through the unit. A humidifier element is shown with reference 11 and a housing with reference number 12. Reference number 13 shows a mesh structure supporting the filter element 9.

Furthermore, the filter element can also be parabolic or hemispherical in its shape. This way a large filtering area with high filtering capacity and low flow resistance can be achieved without increasing the diameter of the filter housing. In addition, the products become lighter and more usable near the patient's facial area. The filter element and the housing of this invention may for example be formed of polypropylene.

In this invention, the ratio between filtering area and housing diameter can be increased up to triple in the cylinder filters (Figure 3) and even quadruple in the plane filters at an oblique angle (Figure 5) compared to the ratio in the conventional breathing circuit filters (Figure 2).

The ratio between the housing 12 diameter and the respiratory tube 5 diameter gives an impression of the dimensions for this invention. When using a single respiratory tube, a 22 mm diameter tube is desirable for adult use, and a 15 mm for pediatric use. When a coaxial tube is used, a 25 mm diameter outer tube is preferred. For these filters to function, the ratio between the housing 12 diameter and the respiratory tube diameter varies approximately between 0.2 and 10. In practice, however, a housing 12 with diameter five times smaller than the diameter of the respiratory tube can be difficult to produce. On the other hand, a housing 12 ten times bigger than the respiratory tube near the patient's face is not practical. Hence, that ratio falls more likely between 0.6 and 4. However, considering the practicality and the breathing resistance of these filters in their applications, the probable ratio between the housing diameter and the respiratory tube diameter varies between 0.8 and 3.5.

In other words, for these kinds of systems to function, maximum diameter of the filter housings 12 in the Figures 3 and 5 is defined to 150 mm and minimum to 5 mm. In practice, however, a housing with outer diameter of 150 mm near the patient's face is not practical. On the other hand, a housing with outer diameter of 5 mm might be difficult to produce. Hence, the maximum diameter of the housing would rather be 60 mm that is the diameter of the current filter (HMEF 750). The minimum housing diameter of 15 mm still enables that the length of the housing would be acceptable. However, considering the practicality and the breathing resistance of these systems in their applications, the probable dimensions for the housing diameter varies between 20-50 mm.

The humidifying element 11 of this invention is also modified when compared to the conventional structures. It is no longer a disc either, but anything else as is shown in the Figures 3 - 6, sphere, oblique at one side or many sides, or it may be combinations of cylinders with different radius.

Figure 6 shows an embodiment of the invention using hemispherical humidifier element 11 and a plane filter 9.

In addition, the element may consist of several parts instead of one and the parts may be separated situating in different locations as presented in the Figure 3. The humidifying element may as well be just one part but cut so that it is a combination of for example a cylinder and a cone. The humidifying element of this invention may for example be formed of polyethylene, polypropylene or polyurethane. In the embodiments described above both filter and humidifier elements are used. It is however quite possible within the spirit of the invention to use only a filter element or only a humidifier element in the gas processing unit described.

The filter element 9 of this invention can be backed with a mesh structure 13 presented in the Figures 5 and 7, or other supporting structure that gives the required support to the filter. The supporting mesh structure 13 has larger distance between pores 14 at the center of the structure. As shown in Figures 7 and 8 the distance between the pores 14 gradually decreases towards the edges in order to inhibit air and moisture going straight through the filter center in a narrow sector and not utilizing the whole filtering area. When the moisture from the breathing air is accumulated to a narrow sector in the filter center the flow resistance of the filter increases. This mesh structure 13 improves the filter efficiency because air and moisture are now spreading more equally to the entire filtering area and hence the flow resistance of the filter remains low. The same structure that supports filter element 9 will support the humidifier element 11 as well, if required. In most cases, however, the outer shell or the geometry of the element itself provides adequate support for the humidifier 11.

Either the filter element 9 or the humidifying element 11 or both can be used in patient breathing circuits. If they both are used at the same time, the humidifier element 11 always situates at the distal end DE, i.e. patient end of the breathing circuit. When the filter element 9 is shaped as a cylinder or a cone, the humidifier element 11 might be wrapped around the filter element or vice versa (Figs. 3 and 4). The supporting mesh (Fig. 7) might locate in three different positions: it can locate between the filter element and humidifier element, as presented in the Fig. 5, or it can locate either in the proximal end, i.e. at the machine end of the filter element, or in the distal end of the humidifier element.

These types of filter and humidifier elements will decrease the diameter of filter housings by using the space more effectively, are lighter in weight and more usable near the patient's facial area, and still have even better properties than the conventional products.

The embodiments described above are by no means intended to restrict the invention, but the invention can be modified completely freely within the scope of the claims. The unit need not be materialized exactly in the way as described in the Figures but the entire structure or its details can be formed otherwise too.

## Claims

1. Gas processing unit in patient circuits for use in anaesthesia or respiratory care, the patient circuit comprising a gas source (1,2) for creating gas mixture for patient breathing and circulating arrangement (3,4,5,6) for delivering the gas mixture to the patient (7), the circulating arrangement comprising tubes (5) through which gases flow to the patient and from the patient, and the circulating arrangement having a proximal end (PE) connected to the gas source for creating gas mixture, and a distal end (DE) connected to the patient (7), and a processing unit (8), the processing unit (8) comprising a housing and at least one processing element placed in the housing, **characterized in that** the processing element (9,11) is arranged to form an angle other than 90 degrees with respect to a direction of gas flow in the tubes (5).

2. Gas processing unit according to claim 1, **characterized in that** the angle is between 2 - 68 degrees.

3. Gas processing unit according to claim 2, **characterized in that** the angle is between 7,5 - 60 degrees.

4. Gas processing unit according to claim 1, **characterized in that** ratio between the housing (12) diameter and the tube (5) diameter is between the values 0,8 and 3,5.

5. Gas processing unit according to claim 1, **characterized in that** the housing diameter (12) varies between the values 20 - 50 mm.

6. Gas processing unit according to claim 1, **characterized in that** the processing element is a filter element (9).

7. Gas processing unit according to claim 1, **characterized in that** the processing element is a humidifier element (11).

8. Gas processing unit according to claim 1, **characterized in that** the processing element comprises a filter element (9) and a humidifier element (11).

9. Gas processing unit according to any of the claims 6 - 8, **characterized in that** the filter or/and humidifier elements (9,11) are shaped as cylinders.

10. Gas processing unit according to claim 6 or 8, **characterized in that** the filter element (9) is wrapped around a supporting frame (10).

11. Gas processing unit according to any of the claims 6 - 8, **characterized in that** the filter or/and humidifier elements (9,11) are shaped as cones.

12. Gas processing unit according to claim 11, **characterized in that** the filter element (9) is wrapped around a supporting frame (10).

13. Gas processing unit according to any of the claims 6 or 8, **characterized in that** the filter element (9) is a plane element.

14. Gas processing unit according to claims 7 or 8, **characterized in that** the humidifier element (11) is formed of one or more elements.

15. Gas processing unit according to claim 13, **characterized in that** the filter element (9) is supported by a mesh structure (13).

16. Gas processing unit according to claim 14, **characterized in that** the humidifier element (11) is a hemispherical element.

17. Gas processing unit according to claim 15, **characterized in that** the mesh structure (13) has larger distance between pores (14) at the centre of the structure and the distance between the pores (14) gradually degreases towards the edges of the mesh structure (13).

18. Gas processing unit according to claim 1, **characterized in that** the processing unit (8) is placed at a distal end (DE) of the patient circuit.
